# EUROPEAN PATENT APPLICATION

(11) **EP 4 541 901 A1**
(43) Date of publication of application: **23.04.2025**
(21) Application number: 23854037.1
(22) Date of filing: 06.06.2023
(51) Int. Cl.: C12P 1/02, C12N 1/14

(54) **ORGANIC SOLID-STATE FERMENTATION ENZYME FORMULATION AND ORGANIC PLANT PROTEASE HYDROLYSATE, AND PREPARATION METHODS THEREFOR**

(30) Priority: 15.08.2022 CN 202210974114
(71) Applicant: Angel Yeast Co., Ltd, Yichang, Hubei 443003 (CN)
(72) Inventor: QIN, Xianwu, Yichang, Hubei 443003 (CN); ZHENG, Ping, Yichang, Hubei 443003 (CN); ZHANG, Yan, Yichang, Hubei 443003 (CN); CHEN, Hui, Yichang, Hubei 443003 (CN); YAO, Hong, Yichang, Hubei 443003 (CN); LIU, Xiuji, Yichang, Hubei 443003 (CN); HE, Guoqing, Yichang, Hubei 443003 (CN); YU, Can, Yichang, Hubei 443003 (CN)
(74) Representative: karo IP
(86) International application number: PCT/CN2023/098472
(87) International publication number: WO 2024/037131

(57) **Abstract**

An organic solid-state fermentation enzyme formulation and an organic plant protease hydrolysate, and preparation methods therefor. The organic solid-state fermentation enzyme formulation is prepared by solid-state fermentation of protease-producing microorganisms, wherein the organic solid-state fermentation enzyme formulation comprises neutral protease, acidic protease, alkaline protease, glucoamylase, and cellulase. By means of using the protease-producing microorganisms to perform solid-state fermentation on organic plant raw materials, the organic solid-state fermentation enzyme formulation rich in the acidic protease, the neutral protease, the alkaline protease, the cellulase, and the glucoamylase is obtained, and then a small amount of additional enzyme is added in the organic solid-state fermentation enzyme formulation, thereby improving the plant proteolysis efficiency by means of an enzyme system synergistic complementary effect.

## Description

### Technical Field

The present invention relates to the field of biotechnology, and in particular, to an organic solid-state fermentation enzyme formulation and an organic plant protease hydrolysate, and preparation methods therefor.

### Background Art

Food safety has always been a general concern of the government and society. With the improvement of people's living standards, there is an increasing requirement for agricultural, fermented, and health products. The popularity of "organic fruits and vegetables," "organic enzymes," "organic products," and the like in the market has been rising, and they are loved by many people. Organic-certified products have a broad international development space. Based on the concept of "returning to nature and protecting the environment," organic products, as the future trend of the international market, have been highly sought after by many brand companies. In recent years, the increasing demand for organic fermented products has driven the development of organic-certified nitrogen sources in the fermentation industry. Plant proteins are important organic-certified nitrogen sources for biological fermentation. At present, due to the high price of plant protein raw materials that meet organic certification and the low efficiency of existing proteolysis, the production cost of organic nitrogen sources is a serious problem. Moreover, enzymatic hydrolysis requires the addition of a large number of biological enzymes, as well as acids and alkalis, to control pH, making it difficult for the hydrolysates to pass the organic certification. Therefore, there is a need for a process that can efficiently hydrolyze plant proteins and meet organic certification requirements to solve the existing problems in organic nitrogen source production.

### Summary of the Invention

The technical problem to be solved by the present invention is to provide an organic solid-state fermentation enzyme formulation, which can improve proteolysis efficiency and reduce the production cost of organic plant protease hydrolysate.

In view of the shortcomings of the prior art, it is an object of the present invention to provide an organic solid-state fermentation enzyme formulation. It is a second object of the present invention to provide a preparation method for the above organic solid-state fermentation enzyme formulation. It is a third object of the present invention to provide a preparation method for an organic plant protease hydrolysate. It is a fourth object of the present invention to provide an organic plant protease hydrolysate prepared by the above preparation method. It is a fifth object of the present invention to provide the use of the organic solid-state fermentation enzyme formulation or the organic plant protease hydrolysate as described above.

The present invention has the following technical solutions.

The present invention provides an organic solid-state fermentation enzyme formulation. The organic solid-state fermentation enzyme formulation is prepared by solid-state fermentation of protease-producing microorganisms, wherein the organic solid-state fermentation enzyme formulation comprises neutral protease, acidic protease, alkaline protease, glucoamylase, and cellulase.

Preferably, in the above organic solid-state fermentation enzyme formulation, the enzyme activity of the acidic protease is >390 u/g, the enzyme activity of the neutral protease is >1500 u/g, the enzyme activity of the alkaline protease is >600 u/g, the enzyme activity of the glucoamylase is >400 u/g, and the enzyme activity of the cellulase is >130 u/g.

Preferably, in the above organic solid-state fermentation enzyme formulation, the enzyme activity of the acidic protease is >800 u/g, the enzyme activity of the neutral protease is >4000 u/g, the enzyme activity of the alkaline protease is >1000 u/g, the enzyme activity of the glucoamylase is >1500 u/g, and the enzyme activity of the cellulase is >300 u/g.

Preferably, in the above organic solid-state fermentation enzyme formulation, the enzyme activity of the acidic protease is >1200 u/g, the enzyme activity of the alkaline protease is >2200 u/g, and the enzyme activity of the cellulase is >1200 u/g.

Preferably, in the above organic solid-state fermentation enzyme formulation, the protease-producing microorganisms comprise one or a combination of two or more selected from the group consisting of *Aspergillus oryzae, Aspergillus niger,* and *Bacillus sp.,* preferably *Aspergillus oryzae* and *Bacillus sp.;* preferably, the weight ratio of *Aspergillus oryzae* to *Bacillus sp.* is (70-90):(10-30).

The present invention further provides a preparation method for the above organic solid-state fermentation enzyme formulation, comprising the steps of:
(1) performing seed culture of protease-producing microorganisms to obtain solid-state seeds; and
(2) performing solid-state fermentation of the solid-state seeds obtained in step (1) to obtain an organic solid-state fermentation enzyme formulation.

Preferably, in the preparation method for the above organic solid-state fermentation enzyme formulation, the solid-state fermentation is solid-state temperature shift fermentation, which is first carried out at 36-40°C for 6-20 h and then at 28-32°C for 30-50 h; preferably, the solid-state temperature shift fermentation is first carried out at 36-38°C for 8-15 h and then at 30-32°C for 40-44 h.

Preferably, in the preparation method for the above organic solid-state fermentation enzyme formulation, both the seed medium in step (1) and the solid-state fermentation medium in step (2) contain organic starch agricultural and sideline products and/or organic oil agricultural and sideline products; preferably, both the seed medium in step (1) and the solid-state fermentation medium in step (2) contain organic starch agricultural and sideline products and organic oil agricultural and sideline products; more preferably, the content of the organic starch agricultural and sideline products is 9-40% by weight, and the content of the organic oil agricultural and sideline products is 60-91% by weight.

Preferably, the organic starch agricultural and sideline products comprise one or a combination of two or more selected from the group consisting of organic rice, organic wheat, and organic corn, preferably organic corn.

Preferably, the organic oil agricultural and sideline products comprise organic bean cake and/or organic rapeseed cake, preferably organic bean cake.

Preferably, in the preparation method for the above organic solid-state fermentation enzyme formulation, both the seed medium in step (1) and the solid-state fermentation medium in step (2) further contain organic plant protein powder; preferably, the organic plant protein powder is 6-10%, and the organic starch agricultural and sideline products and/or organic oil agricultural and sideline products is 90-94%, based on the total weight of 100% of the medium.

Preferably, in the preparation method for the above organic solid-state fermentation enzyme formulation, the organic plant protein powder comprises organic rice protein powder and/or organic wheat protein powder; further preferably, the organic plant protein powder comprises 60-80% by weight of organic rice protein powder and 20-40% by weight of organic wheat protein powder.

Preferably, in the preparation method for the above organic solid-state fermentation enzyme formulation, the inoculation amount for the seed culture in step (1) is 0.3-0.5% based on the weight of the seed medium.

Preferably, in the preparation method for the above organic solid-state fermentation enzyme formulation, the inoculation amount for the solid-state fermentation in step (2) is 0.3-0.5% based on the weight of the solid-state fermentation medium.

Preferably, the preparation method for the above organic solid-state fermentation enzyme formulation further comprises the step of drying the organic solid-state fermentation enzyme formulation; preferably, the drying temperature is 37-40°C.

The present invention further provides a preparation method for an organic plant protease hydrolysate, comprising hydrolyzing enzymatically the above organic solid-state fermentation enzyme formulation or an organic solid-state fermentation enzyme formulation obtained by the preparation method for the above organic solid-state fermentation enzyme formulation using an additional enzyme.

Preferably, in the above preparation method for an organic plant protease hydrolysate, the additional enzyme is used in an amount of 0.2-0.8% based on the dry matter weight of the organic solid-state fermentation enzyme formulation; preferably, the additional enzyme comprises one or a combination of two or more selected from the group consisting of papain, bromelain, neutral protease, acidic protease, alkaline protease, glucanase, and cellulase; more preferably, the additional enzyme comprises 0.1-0.3% papain, 0.04-0.08% neutral protease, 0.02-0.04% acidic protease, 0.01-0.02% glucanase, and 0.01-0.02% cellulase, based on the dry matter weight of the organic solid-state fermentation enzyme formulation.

Preferably, in the above preparation method for an organic plant protease hydrolysate, the enzymatic hydrolysis temperature is 45-55°C, and the enzymatic hydrolysis time is 10-15 h; preferably, the enzymatic hydrolysis pH is between 4.5 and 7.5.

Preferably, the above preparation method for an organic plant protease hydrolysate further comprises the step of refining the organic solid-state fermentation enzyme formulation before the enzymatic hydrolysis; preferably, the dry matter content of a slurry during refining is 12-16% by weight.

Preferably, the above preparation method for an organic plant protease hydrolysate further comprises the steps of sequentially filtering, concentrating, and drying the enzymatic hydrolysate. The present invention further provides an organic plant protease hydrolysate obtained by the above preparation method for an organic plant protease hydrolysate; preferably, the total nitrogen content is >9%, and the amino acid nitrogen content is >4.5%, based on the dry matter weight of the organic plant protease hydrolysate.

The present invention further provides the use of an organic plant protease hydrolysate obtained by the above preparation method for an organic plant protease hydrolysate or the above organic plant protease hydrolysate as an organic nitrogen source in the fermentation field of yeast, lactic acid bacteria, or *Bacillus sp.*

The present invention further provides the use of an organic plant protease hydrolysate obtained by the above preparation method or the above organic plant protease hydrolysate as an organic tasty agent or seasoning base in the field of food seasoning.

The present invention further provides the use of the above organic solid-state fermentation enzyme formulation or an organic solid-state fermentation enzyme formulation obtained by the preparation method for the above organic solid-state fermentation enzyme formulation as an organic protease for the enzymatic hydrolysis of plant proteins.

The present invention has the following beneficial effects.

According to the present invention, by means of using the protease-producing microorganisms to perform solid-state fermentation on organic plant raw materials, the organic solid-state fermentation enzyme formulation rich in the acidic protease, the neutral protease, the alkaline protease, the cellulase, and the glucoamylase is obtained, and then a small amount of additional enzyme is added in the organic solid-state fermentation enzyme formulation, thereby greatly improving the plant proteolysis efficiency by means of an enzyme system synergistic complementary effect, resulting in an amino nitrogen conversion rate of organic plant proteins exceeding 43%. In addition, the prepared organic plant protease hydrolysate can be used as an organic nitrogen source and a tasty agent, which fully meets the EU organic certification requirements and increases product-added values.

### Strain deposit information

The strain *Bacillus subtilis (Bacillus subtilis* ESP710) used in the present invention was deposited at the China Center for Type Culture Collection (CCTCC) on Nov. 26, 2018, with the deposit number of CCTCC NO: M 2018827 (i.e., CCTCC M 2018827), and the deposited address of Wuhan University, Wuhan, China, 430072; phone: (027)-68754052 (the *Bacillus subtilis* has been described in Chinese patent application number 201910686571.8 (publication number CN112300953A)).

The *Aspergillus niger* strain ESP1023 *(Aspergillus niger* ESP1023) used in the present invention was deposited at the China Center for Type Culture Collection (CCTCC) on Nov. 26, 2018, with the address of Wuhan University, Wuhan, China, 430072; phone: 027-68754052, and the deposit number of CCTCC NO: M 2018829 (i.e., CCTCC M 2018829) (the *Aspergillus niger* strain has been described in Chinese patent application number 201911268295.X (publication number CN112940943A)).

### Detailed Description of the Invention

In order to clarify the objects, technical solutions, and technical effects of the embodiments of the present invention, the technical solutions of the embodiments of the present invention are described clearly and completely. The embodiments described below are merely some rather than all of the embodiments of the present invention. All other embodiments obtained by a person of ordinary skill in the art without inventive efforts, combined with the embodiments of the present invention, shall fall within the protection scope of the present invention.

At present, there are two main problems in the production of plant protease hydrolysates that meet organic certification. Firstly, the plant proteolysis technologies need to be improved, and there is no efficient synergistic effect of biological enzymes, resulting in a low enzymatic hydrolysis efficiency, amino nitrogen conversion rate of about 30%, and high production cost of products. Secondly, the enzymatic hydrolysis process of plants requires the addition of a large number of various biological enzymes, as well as strong acids and alkalis to control pH, making it difficult for the process to meet the organic certification requirements.

In a specific embodiment of the present invention, the present invention provides an organic solid-state fermentation enzyme formulation, the organic solid-state fermentation enzyme formulation being prepared by solid-state fermentation of protease-producing microorganisms, wherein the organic solid-state fermentation enzyme formulation comprises neutral protease, acidic protease, alkaline protease, glucoamylase, and cellulase.

In a preferred embodiment of the present invention, in the above organic solid-state fermentation enzyme formulation, the enzyme activity of the acidic protease is >390 u/g, the enzyme activity of the neutral protease is >1500 u/g, the enzyme activity of the alkaline protease is >600 u/g, the enzyme activity of the glucoamylase is >400 u/g, and the enzyme activity of the cellulase is >130 u/g.

Further, in the above organic solid-state fermentation enzyme formulation, the enzyme activity of the acidic protease is >800 u/g, the enzyme activity of the neutral protease is >4000 u/g, the enzyme activity of the alkaline protease is >1000 u/g, the enzyme activity of the glucoamylase is >1500 u/g, and the enzyme activity of the cellulase is >300 u/g.

Still further, in the above organic solid-state fermentation enzyme formulation, the enzyme activity of the acidic protease is >1200 u/g, the enzyme activity of the alkaline protease is >2200 u/g, and the enzyme activity of the cellulase is >1200 u/g.

The protease-producing microorganisms comprise one or a combination of two or more selected from the group consisting of *Aspergillus oryzae, Aspergillus niger,* and *Bacillus sp.,* preferably *Aspergillus oryzae* and *Bacillus sp.;* preferably, the weight ratio of *Aspergillus oryzae* to *Bacillus sp.* is (70-90):(10-30).

The present invention further provides a preparation method for the above organic solid-state fermentation enzyme formulation, comprising the steps of:
(1) performing seed culture of protease-producing microorganisms to obtain solid-state seeds; and
(2) performing solid-state fermentation of the solid-state seeds obtained in step (2) to obtain an organic solid-state fermentation enzyme formulation.

Both the seed medium in step (1) and the solid-state fermentation medium in step (2) contain organic starch agricultural and sideline products and/or organic oil agricultural and sideline products. For example, the organic starch agricultural and sideline products may be one or a combination of two or more selected from the group consisting of organic rice, organic wheat, and organic corn, and the organic oil agricultural and sideline products may be organic bean cake and/or organic rapeseed cake.

In a preferred embodiment of the present invention, both the seed medium in step (1) and the solid-state fermentation medium in step (2) contain organic starch agricultural and sideline products and organic oil agricultural and sideline products. The content of the organic starch agricultural and sideline products is 9-40% by weight, and the content of the organic oil agricultural and sideline products is 60-91% by weight.

Both the seed medium in step (1) and the solid-state fermentation medium in step (2) further contain organic plant protein powder. The organic plant protein powder may be organic rice protein powder and/or organic wheat protein powder; preferably, the organic plant protein powder comprises 60-80% by weight of organic rice protein powder and 20-40% by weight of organic wheat protein powder.

In the seed medium and the solid-state fermentation medium, the organic plant protein powder is 6-10%, and the organic starch agricultural and sideline products and/or organic oil agricultural and sideline products is 90-94%, based on the total weight of 100% of the medium.

The organic rice protein powder and/or the organic wheat protein powder have high protein content and are mostly alkali-soluble proteins, which are beneficial to stimulate strains to secrete a large number of neutral and alkaline proteases, accelerate the enzymatic hydrolysis of protein structures in plants, and significantly improve the conversion efficiency of amino acid nitrogen.

In another preferred embodiment of the present invention, both the seed medium in step (1) and the solid-state fermentation medium in step (2) contain 70-90% organic bean cake granules and 10-30% organic corn granules.

The solid-state fermentation in step (2) is solid-state temperature shift fermentation, wherein the solid-state temperature shift fermentation comprises first fermenting at 36-40°C for 6-20 h and then at 28-32°C for 30-50 h; preferably, the solid-state temperature shift fermentation comprises first fermenting at 36-38°C for 8-15 h and then at 28-30°C for 40-44 h. By means of the solid-state shift temperature fermentation, the enzyme activity of the organic solid-state fermentation enzyme formulation and the conversion rate of amino acid nitrogen in plant proteins can be further improved.

In yet another preferred embodiment of the present invention, the preparation method for the above organic solid-state fermentation enzyme formulation comprises the steps of:
(1) adding protease-producing microorganism strains to 10 mL of sterile water to prepare a spore suspension with a spore concentration of 10⁷-10⁹ cfu/mL, inoculating the spore suspension into a seed medium at an inoculation amount of 0.3-0.5% (based on the weight of the medium), and culturing at 32-38°C for 2-4 d to obtain protease-producing microorganism solid-state seeds;
(2) feeding the protease-producing microorganism solid-state seeds obtained in step (1) into a solid-state fermentation medium in a mixing inoculation machine at an inoculation amount of 0.3-0.5% (based on the weight of the medium), and finally sending it into a koji-making disc machine for koji-making culture, performing regular ventilation and turning over the culture, controlling the ambient temperature at 36-40°C and the humidity at 90-95% for fermentation for 6-20 h, and controlling the ambient temperature at 28-32°C and the humidity at 90-95% for fermentation for 30-50 h after the product temperature rises; and
(3) low-temperature drying the above solid-state fermentation product at 37-40°C to obtain an organic solid fermentation enzyme formulation.

The production method for the seed medium and the solid-state fermentation medium of the present invention comprises the steps of: weighing each raw material according to the formula, adding 85-100% (based on the total weight of raw materials) of water, mixing uniformly, and sterilizing at 121°C for 50 min.

The present invention further provides a preparation method for an organic plant protease hydrolysate, comprising hydrolyzing enzymatically the above organic solid-state fermentation enzyme formulation or an organic solid-state fermentation enzyme formulation obtained by the above preparation method using an additional enzyme.

The "additional enzyme" of the present invention refers to an enzyme from other sources added to the organic solid-state fermentation enzyme formulation, such as one or a combination of two or more selected from the group consisting of papain, bromelain, neutral protease, acidic protease, alkaline protease, glucanase, and cellulase; preferably including one or a combination of two or more selected from the group consisting of papain, acidic protease, neutral protease, glucanase, and cellulase; more preferably including papain, acidic protease, neutral protease, glucanase, and cellulase; preferably, the additional enzyme is used in an amount of 0.2-0.8% based on the dry matter weight of the organic solid-state fermentation enzyme formulation; further preferably, the additional enzyme include 0.1-0.3% papain, 0.04-0.08% neutral protease, 0.02-0.04% acidic protease, 0.01-0.02% glucanase, and 0.01-0.02% cellulase. Since the core of plant proteolysis is the synergistic effect of various biological enzymes, the additional compound enzyme is mainly used further to enrich the enzyme system of proteases and cellulase, thereby enhancing the synergistic effect. For example, papain, as an endopeptidase, can effectively compensate for the shortcomings of the solid-state koji-making endonuclease system.

In the above preparation method for an organic plant protease hydrolysate, the enzymatic hydrolysis temperature is 45-55°C, and the enzymatic hydrolysis time is 10-15 h; preferably, the enzymatic hydrolysis pH is between 4.5 and 7.5.

The pH regulator for the enzymatic hydrolysis is citric acid and/or sodium carbonate.

The above preparation method for an organic plant protease hydrolysate further comprises the step of refining the organic solid-state fermentation enzyme formulation before the enzymatic hydrolysis; preferably, the dry matter content of a slurry during refining is 12-16% by weight. The above preparation method for an organic plant protease hydrolysate further comprises the steps of sequentially filtering, concentrating, and drying the enzymatic hydrolysate.

In a preferred embodiment of the present invention, the above preparation method for an organic plant protease hydrolysate further comprises the steps of:
(1) adding water to the above organic solid-state fermentation enzyme formulation to prepare a reaction solution with a dry matter mass concentration of 12-16%, refining using a colloid mill, adding 0.2-0.8% of an additional enzyme based on the dry matter weight of the reaction solution for enzymatic hydrolysis, wherein the enzymatic hydrolysis temperature is 45-55°C, the enzymatic hydrolysis pH is 4.5-7.5, and the enzymatic hydrolysis time is 10-15 h;
(2) adding perlite to the above enzymatic hydrolysate solution, mixing uniformly, performing plate-frame press filtration to obtain a filtrate, distilling and concentrating under reduced pressure the filtrate to obtain a concentrated solution with a dry matter of 25-35%, and finally adding organic maltodextrin with a concentrated solution mass fraction of 5-10%, stirring for dissolution, and then spray drying to obtain an organic plant protease hydrolysate.

The present invention further provides an organic plant protease hydrolysate obtained by the above preparation method, wherein the total nitrogen content is >9%, and the amino acid nitrogen content is >4.5%, based on the dry matter weight of the organic plant protease hydrolysate. According to the present invention, by means of using the protease-producing microorganisms to perform solid-state temperature shift fermentation, a fermentation product rich in the acidic protease, the neutral protease, the alkaline protease, the cellulase, and the glucoamylase is obtained, and a small amount of additional enzyme is added, thereby greatly improving the plant proteolysis efficiency by means of an enzyme system synergistic complementary effect. Chemical substances such as hydrochloric acid, sulfuric acid, and sodium hydroxide are basically not added throughout the entire process, which can meet the requirement of external non-organic certified substance content to be <5% and thus meet the EU organic certification requirements.

The present invention further provides the use of the above organic plant protease hydrolysate as an organic nitrogen source in the fermentation field of yeast, lactic acid bacteria, or *Bacillus sp.* The present invention further provides the use of the above organic plant protease hydrolysate as an organic tasty agent or seasoning base in the field of food seasoning.

The present invention further provides the use of the above organic solid-state fermentation enzyme formulation as an organic protease for the enzymatic hydrolysis of plant proteins.

The beneficial effects of the organic solid-state fermentation enzyme formulation and the organic plant protease hydrolysate, and preparation methods therefor, according to the present invention are further illustrated by the following specific examples.

The raw materials and equipment sources used in the inventive and comparative examples are shown in Table 1.

**Table 1. Raw materials used in the inventive and comparative examples**

| Raw material or equipment used | Model \ Purity | Vendor |
|---|---|---|
| Organic bean cake | >95% | JiYuan Organic Grain & Oil Co., Ltd. |
| Organic rapeseed cake | >95% | Anhui Fujia Oil Factory Oil Industry Co., Ltd. |
| Organic corn | >95% | Fujin Fuhui Food Technology Co., Ltd. |
| Organic wheat | >95% | Heilongjiang Hongmao Agricultural Development Co., Ltd. |
| Organic wheat protein powder | Protein content >80% | Fengqiu County Huafeng Powder Industry Co., Ltd. |
| Organic rice | >95% | Inner Mongolia Keqin Wanjia Food Co., Ltd. |
| Organic rice protein powder | Protein content >82% | Anhui Shunxin Shengyuan Biological Food Co., Ltd. |
| Angel compound protease | Enzyme activity >60000 U/g | Angel Enzyme Preparation Co., Ltd. |
| Papain | Enzyme activity >60000 U/g | Angel Enzyme Preparation Co., Ltd. |
| Bromelain | Enzyme activity >100000 u/g | Nanning Pangbo Biological Engineering Co., Ltd. |
| *Aspergillus oryzae* strain | Spores >20 billion/g | Hubei Qiming Biotechnology Co., Ltd. |

### Example 1

### (I) Preparation of solid-state seeds

80 g of organic bean cake granules and 20 g of organic corn granules were weighed, and 8 g of composite organic plant protein powder (composed of 80 wt% organic rice protein powder and 20 wt% organic wheat protein powder) was added. Then, 90 g of water was added, mixed uniformly, and sterilized at 120°C for 50 min to obtain a solid-state seed medium.

An *Aspergillus oryzae* strain preserved on a slope was added to 10 mL of sterile water to prepare a spore suspension with a spore concentration of 10⁷-10⁸ cfu/ml. The spore suspension was inoculated into the solid-state seed medium at an inoculation amount of 0.3% (based on the weight of the solid-state seed medium) and cultured at 32°C for 3 d to obtain *Aspergillus oryzae* solid-state seeds.

### (II) Preparation of an organic solid-state fermentation enzyme formulation

1000 g of organic bean cake granules with a 6-14 mesh granule size were weighed, and 80 g of composite organic plant protein powder (composed of 60 wt% organic rice protein powder and 40 wt% organic wheat protein powder) was added. Then, 950 g of water was added, mixed uniformly, and sterilized at 121°C for 50 min to obtain a solid-state fermentation medium.

The *Aspergillus oryzae* solid-state seeds were inoculated into the above solid-state fermentation medium at an inoculation amount of 0.4% (based on the weight of the medium), and sent into a koji-making disc machine for koji-making culture. Regular ventilation and turning over the culture were carried out. The ambient temperature was controlled at 36°C, and the humidity was controlled at 94% for high-temperature fermentation for 15 h. After the product temperature rose, the ambient temperature was controlled at 30°C, and the humidity was controlled at 90% for low-temperature fermentation for 44 h. Finally, low-temperature drying was carried out at 38°C to obtain an organic solid-state fermentation enzyme formulation rich in various biological enzymes. The enzymatic activities of proteases, glucoamylase, and cellulase in the organic solid-state fermentation enzyme formulation were determined by the following method. The results are shown in Table 2.

### 1. Determination of the enzyme activity of proteases

The determination was carried out according to the method specified in Annex B (Determination of protease activity: Folin method) of GB/T 23527-2009 (Protease formulations). Phosphate buffer was used to determine the enzyme activity of neutral protease, sodium lactate buffer was used to determine the enzyme activity of acidic protease, and boric acid buffer solution was used to determine the enzyme activity of alkaline protease.

### 2. Determination of the enzyme activity of glucoamylase

The determination was carried out according to the method specified in 5.6 Saccharogenic power of QB/T 4527-2011 (General analytical method for Daqu brewing).

### 3. Determination of the enzyme activity of cellulase

The determination was carried out according to the method specified in Annex A (Determination method for the enzyme activity in filter paper) of QB/T 2583-2003 (Cellulase formulations).

### (III) Preparation of an organic plant protease hydrolysate

Water was added to the organic solid-state fermentation enzyme formulation obtained in the above step (II) to prepare a solution with a dry matter concentration of 16 wt%. The solution was refined into emulsion using a colloid mill, and 0.2% Angel compound protease and 0.3% papain were added, based on the dry matter weight, for enzymatic hydrolysis at a temperature of 50°C. The pH was maintained between 5.0 and 6.5 using a sodium carbonate solution. The enzymatic hydrolysis was carried out for 12 h to obtain an organic plant protease hydrolysate solution. The Angel compound protease included 40% by weight of neutral protease, 20% by weight of acidic protease, 10% by weight of glucanase, and 10% by weight of cellulase.

Perlite was added to the above organic plant protease hydrolysate solution, mixed uniformly, and then filtered using a plate-and-frame filter press to obtain a filtrate. The filtrate was concentrated under reduced pressure to obtain a concentrated solution with a dry matter concentration of 25%. Finally, 10 wt% organic maltodextrin (based on the mass of the concentrated solution) was added to the concentrated solution, stirred for dissolution, and then spray dried to obtain an organic plant protease hydrolysate.

The total nitrogen content and amino acid nitrogen content of the organic plant protease hydrolysate solution and the organic plant protease hydrolysate were determined by the following methods. The conversion rate of amino acid nitrogen in the organic plant protease hydrolysate solution was used as a measurable indicator of enzymatic hydrolysis efficiency. The results are shown in Table 3.

### 1. Determination of total nitrogen content

Total nitrogen was determined according to the method specified in Section 3: Combustion method of GB 5009.5-2016 (Determination of proteins in food).

### 2. Determination of amino acid nitrogen content

The determination was carried out according to 5.2 Sauce and soybean sauce samples in the first method (acidimeter method) of GB 5009.235-2016 (Determination of amino acid nitrogen in food).

### 3. Conversion rate of amino acid nitrogen

Conversion rate of amino acid nitrogen = amino acid nitrogen content of enzymatic hydrolysate solution / total nitrogen content of enzymatic hydrolysate solution * 100%

### Example 2

### (I) Preparation of solid-state seeds

70 g of organic bean cake granules and 30 g of organic corn granules were weighed, and 8 g of composite organic plant protein powder (composed of 80 wt% organic rice protein powder and 20 wt% organic wheat protein powder) was added. Then, 100 g of water was added, mixed uniformly, and sterilized at 120°C for 50 min to obtain a solid-state seed medium.

*A Bacillus subtilis* (*Bacillus subtilis* ESP710) strain preserved on a slope was added to 10 mL of sterile water to prepare a bacterial solution with a *Bacillus* concentration of 10⁸-10⁹ cfu/ml. The *Bacillus subtilis* was inoculated into the solid-state seed medium at an inoculation amount of 0.1% (based on the weight of the medium) and cultured at 38°C for 2 d to obtain *Bacillus subtilis* solid-state seeds.

### (II) Preparation of an organic solid-state fermentation enzyme formulation

1000 g of organic bean cake granules with a 6-14 mesh granule size were weighed, and 80 g of composite organic plant protein powder (composed of 60 wt% organic rice protein powder and 40 wt% organic wheat protein powder) was added. Then, 950 g of water was added, mixed uniformly, and sterilized at 121°C for 50 min to obtain a solid-state fermentation medium.

The *Bacillus* solid-state seeds were inoculated into the above solid-state fermentation medium at an inoculation amount of 0.4% (based on the weight of the medium), and sent into a koji-making disc machine for koji-making culture. Regular ventilation and turning over the culture were carried out. The ambient temperature was controlled at 40°C, and the humidity was controlled at 95% for high-temperature fermentation for 20 h. After the product temperature rose, the ambient temperature was controlled at 28°C, and the humidity was controlled at 90% for low-temperature fermentation for 50 h. Finally, low-temperature drying was carried out at 38°C to obtain an organic solid-state fermentation enzyme formulation rich in various biological enzymes.

The enzymatic activities of proteases, glucoamylase, and cellulase in the organic solid-state fermentation enzyme formulation were determined by the methods in Example 1. The results are shown in Table 2.

### (III) Preparation of an organic plant protease hydrolysate

Water was added to the organic solid-state fermentation enzyme formulation obtained in the above step (II) to prepare a solution with a dry matter concentration of 16%. The solution was refined into emulsion using a colloid mill, and 0.2% papain was added, based on the dry matter weight, for enzymatic hydrolysis at a temperature of 55°C. The pH was maintained between 4.5 and 5.0 using a citric acid solution. The enzymatic hydrolysis was carried out for 15 h to obtain an organic plant protease hydrolysate solution.

Perlite was added to the above organic plant protease hydrolysate solution, mixed uniformly, and then filtered using a plate-and-frame filter press to obtain a filtrate. The filtrate was concentrated under reduced pressure to obtain a concentrated solution with a dry matter concentration of 25%. Finally, 10 wt% organic maltodextrin (based on the mass of the concentrated solution) was added to the concentrated solution, stirred for dissolution, and then spray dried to obtain an organic plant protease hydrolysate.

The total nitrogen content and amino acid nitrogen content of the organic plant protease hydrolysate solution and the plant protease hydrolysate were determined by the methods in Example 1. The conversion rate of amino acid nitrogen in the organic plant protease hydrolysate solution was used as a measurable indicator of enzymatic hydrolysis efficiency. The results are shown in Table 3.

### Example 3

### (I) Preparation of solid-state seeds

90 g of organic bean cake and 10 g of organic corn were weighed, and 8 g of composite organic plant protein powder (composed of 80 wt% organic rice protein powder and 20 wt% organic wheat protein powder) was added. Then, 95 g of water was added, mixed uniformly, and sterilized at 120°C for 50 min to obtain a solid-state seed medium.

An *Aspergillus niger* strain ESP1023 (*Aspergillus niger* ESP1023) preserved on a slope was added to 10 mL of sterile water to prepare a spore suspension with a spore concentration of 10⁷-10⁸ cfu/ml. The spore suspension was inoculated into the solid-state seed medium at an inoculation amount of 0.3% (based on the weight of the medium) and cultured at 32°C for 3 d to obtain *Aspergillus niger* solid-state seeds.

### (II) Preparation of an organic solid-state fermentation enzyme formulation

1000 g of organic bean cake granules with a 6-14 mesh granule size were weighed, and 80 g of composite organic plant protein powder (composed of 60 wt% organic rice protein powder and 40 wt% organic wheat protein powder) was added. Then, 950 g of water was added, mixed uniformly, and sterilized at 121°C for 50 min to obtain a solid-state fermentation medium.

The *Aspergillus niger* solid-state seeds were inoculated into the above solid-state fermentation medium at an inoculation amount of 0.4% (based on the weight of the medium), and sent into a koji-making disc machine for koji-making culture. Regular ventilation and turning over the culture were carried out. The ambient temperature was controlled at 38°C, and the humidity was controlled at 94% for high-temperature fermentation for 20 h. After the product temperature rose, the ambient temperature was controlled at 30°C, and the humidity was controlled at 90% for low-temperature fermentation for 30 h. Finally, low-temperature drying was carried out at 38°C to obtain an organic solid-state fermentation enzyme formulation rich in various biological enzymes. The enzymatic activities of proteases, glucoamylase, and cellulase in the organic solid-state fermentation enzyme formulation were determined by the methods in Example 1. The results are shown in Table 2.

### (III) Preparation of an organic plant protease hydrolysate

Water was added to the organic solid-state fermentation enzyme formulation obtained in the above step (II) to prepare a solution with a dry matter concentration of 16%. The solution was refined into emulsion using a colloid mill, and 0.8% bromelain was added, based on the dry matter weight, for enzymatic hydrolysis at a temperature of 55°C. The pH was maintained between 7.0 and 7.5 using a sodium carbonate solution. The enzymatic hydrolysis was carried out for 10 h to obtain an organic plant protease hydrolysate solution.

Perlite was added to the above organic plant protease hydrolysate solution, mixed uniformly, and then filtered using a plate-and-frame filter press to obtain a filtrate. The filtrate was concentrated under reduced pressure to obtain a concentrated solution with a dry matter concentration of 35%. Finally, 5 wt% organic maltodextrin (based on the mass of the concentrated solution) was added to the concentrated solution, stirred for dissolution, and then spray dried to obtain an organic plant protease hydrolysate.

The total nitrogen content and amino acid nitrogen content of the organic plant protease hydrolysate solution and the plant protease hydrolysate were determined by the methods in Example 1. The conversion rate of amino acid nitrogen in the organic plant protease hydrolysate solution was used as a measurable indicator of enzymatic hydrolysis efficiency. The results are shown in Table 3.

### Example 4

### (I) Preparation of solid-state seeds

The *Aspergillus oryzae* solid-state seeds obtained in Example 1 and the *Bacillus subtilis* solid-state seeds obtained in Example 2 were mixed at a weight ratio of 90:10 to obtain compound bacteria solid-state seeds.

### (II) Preparation of an organic solid-state fermentation enzyme formulation

1000 g of organic rapeseed cake granules with a 6-14 mesh granule size were weighed, and 80 g of composite organic plant protein powder (composed of 60 wt% organic rice protein powder and 40 wt% organic wheat protein powder) was added. Then, 900 g of water was added, mixed uniformly, and sterilized at 121°C for 50 min to obtain a solid-state fermentation medium.

The compound bacteria solid-state seeds were inoculated into the above solid-state fermentation medium at an inoculation amount of 0.5% (based on the weight of the medium), and sent into a koji-making disc machine for koji-making culture. Regular ventilation and turning over the culture were carried out. The ambient temperature was controlled at 36°C, and the humidity was controlled at 94% for high-temperature fermentation for 15 h. After the product temperature rose, the ambient temperature was controlled at 30°C, and the humidity was controlled at 90% for low-temperature fermentation for 44 h. Finally, drying was carried out at 40°C to obtain an organic solid-state fermentation enzyme formulation rich in various biological enzymes.

The enzymatic activities of proteases, glucoamylase, and cellulase in the organic solid-state fermentation enzyme formulation were determined by the methods in Example 1. The results are shown in Table 2.

### (III) Preparation of an organic plant protease hydrolysate

Water was added to the organic solid-state fermentation enzyme formulation obtained in the above step (II) to prepare a solution with a dry matter concentration of 12%. The solution was refined into emulsion using a colloid mill, and 0.1% Angel compound protease and 0.1% papain were added, based on the dry matter weight, for enzymatic hydrolysis at a temperature of 50°C. The pH was maintained between 5.0 and 5.5 using a sodium carbonate solution. The enzymatic hydrolysis was carried out for 10 h to obtain an organic plant protease hydrolysate solution.

Perlite was added to the organic plant protease hydrolysate solution, mixed uniformly, and then filtered using a plate-and-frame filter press to obtain a filtrate. The filtrate was concentrated under reduced pressure to obtain a concentrated solution with a dry matter concentration of 25%. Finally, 10 wt% organic maltodextrin (based on the mass of the concentrated solution) was added to the concentrated solution, stirred for dissolution, and then spray dried to obtain an organic plant protease hydrolysate.

The total nitrogen content and amino acid nitrogen content of the organic plant protease hydrolysate solution and the plant protease hydrolysate were determined by the methods in Example 1. The conversion rate of amino acid nitrogen in the organic plant protease hydrolysate solution was used as a measurable indicator of enzymatic hydrolysis efficiency. The results are shown in Table 3.

### Example 5

### (I) Preparation of solid-state seeds

The *Aspergillus oryzae* solid-state seeds obtained in Example 1 and the *Bacillus subtilis* solid-state seeds obtained in Example 2 were mixed at a weight ratio of 70:30 to obtain compound bacteria solid-state seeds.

### (II) Preparation of an organic solid-state fermentation enzyme formulation

800 g of organic bean cake granules with a 6-14 mesh granule size and 200 g of organic corn granules with a 10-30 mesh particle size were weighed, and 80 g of composite organic plant protein powder (composed of 60 wt% organic rice protein powder and 40 wt% organic wheat protein powder) was added. Then, 950 g of water was added, mixed uniformly, and sterilized at 121°C for 50 min to obtain a solid-state fermentation medium.

The compound bacteria solid-state seeds were inoculated into the above solid-state fermentation medium at an inoculation amount of 0.3% (based on the weight of the medium), and sent into a koji-making disc machine for koji-making culture. Regular ventilation and turning over the culture were carried out. The ambient temperature was controlled at 38°C, and the humidity was controlled at 95% for high-temperature fermentation for 15 h. After the product temperature rose, the ambient temperature was controlled at 32°C, and the humidity was controlled at 95% for low-temperature fermentation for 44 h. Finally, drying was carried out at 37°C to obtain an organic solid-state fermentation enzyme formulation rich in various biological enzymes.

The enzymatic activities of proteases, glucoamylase, and cellulase in the organic solid-state fermentation enzyme formulation were determined by the following method. The results are shown in Table 2.

### (III) Preparation of an organic plant protease hydrolysate

Water was added to the organic solid-state fermentation enzyme formulation obtained in the above step (II) to prepare a solution with a dry matter concentration of 12%. The solution was refined into emulsion using a colloid mill, and 0.15% compound protease and 0.2% papain were added, based on the dry matter weight, for enzymatic hydrolysis at a temperature of 50°C. The pH was maintained between 5.5 and 6.5 using a sodium carbonate solution. The enzymatic hydrolysis was carried out for 10 h to obtain an organic plant protease hydrolysate solution.

Perlite was added to the organic plant protease hydrolysate solution, mixed uniformly, and then filtered using a plate-and-frame filter press to obtain a filtrate. The filtrate was concentrated under reduced pressure to obtain a concentrated solution with a dry matter concentration of 25%. Finally, 5 wt% organic maltodextrin (based on the mass of the concentrated solution) was added to the concentrated solution, stirred for dissolution, and then spray dried to obtain an organic plant protease hydrolysate.

The total nitrogen content and amino acid nitrogen content of the organic plant protease hydrolysate solution and the plant protease hydrolysate were determined by the methods in Example 1. The conversion rate of amino acid nitrogen in the organic plant protease hydrolysate solution was used as a measurable indicator of enzymatic hydrolysis efficiency. The results are shown in Table 3.

### Example 6

Compared with Example 1, the difference is that neither the solid-state seed medium in step (I) nor the solid-state fermentation medium in step (II) contained plant protein powder. The specific preparation method was as follows:

### (I) Preparation of solid-state seeds

80 g of organic bean cake granules and 20 g of organic corn granules were weighed. Then, 90 g of water was added, mixed uniformly, and sterilized at 120°C for 50 min to obtain a solid-state seed medium.

An *Aspergillus oryzae* strain preserved on a slope was added to 10 mL of sterile water to prepare a spore suspension with a spore concentration of 10⁷-10⁸ cfu/ml. The spore suspension was inoculated into the solid-state seed medium at an inoculation amount of 0.3% (based on the weight of the medium) and cultured at 32°C for 3 d to obtain *Aspergillus oryzae* solid-state seeds.

### (II) Preparation of an organic solid-state fermentation enzyme formulation

1000 g of organic bean cake granules with a 6-14 mesh granule size were weighed. Then, 950 g of water was added, mixed uniformly, and sterilized at 121°C for 50 min to obtain a solid-state fermentation medium.

The *Aspergillus oryzae* solid-state seeds were inoculated into the above solid-state fermentation medium at an inoculation amount of 0.4% (based on the weight of the medium), and sent into a koji-making disc machine for koji-making culture. Regular ventilation and turning over the culture were carried out. The ambient temperature was controlled at 36°C, and the humidity was controlled at 94% for high-temperature fermentation for 15 h. After the product temperature rose, the ambient temperature was controlled at 30°C, and the humidity was controlled at 90% for low-temperature fermentation for 44 h. Finally, low-temperature drying was carried out at 38°C to obtain an organic solid-state fermentation enzyme formulation rich in various biological enzymes.

### (III) Preparation of an organic plant protease hydrolysate

Water was added to the organic solid-state fermentation enzyme formulation obtained in the above step (II) to prepare a solution with a dry matter concentration of 16 wt%. The solution was refined into emulsion using a colloid mill, and 0.2% Angel compound protease and 0.3% papain were added, based on the dry matter weight, for enzymatic hydrolysis at a temperature of 50°C. The pH was maintained between 5.0 and 6.5 using a sodium carbonate solution. The enzymatic hydrolysis was carried out for 12 h to obtain an organic plant protease hydrolysate solution. The Angel compound protease included 40% by weight of neutral protease, 20% by weight of acidic protease, 10% by weight of glucanase, and 10% by weight of cellulase.

Perlite was added to the above organic plant protease hydrolysate solution, mixed uniformly, and then filtered using a plate-and-frame filter press to obtain a filtrate. The filtrate was concentrated under reduced pressure to obtain a concentrated solution with a dry matter concentration of 25%. Finally, 10 wt% organic maltodextrin (based on the mass of the concentrated solution) was added to the concentrated solution, stirred for dissolution, and then spray dried to obtain an organic plant protease hydrolysate.

The enzymatic activities of proteases, glucoamylase, and cellulase in the organic solid-state fermentation enzyme formulation, as well as the total nitrogen content and amino acid nitrogen content of the organic plant protease hydrolysate solution and the plant protease hydrolysate, were determined by the methods in Example 1. The conversion rate of amino acid nitrogen in the organic plant protease hydrolysate solution was used as a measurable indicator of enzymatic hydrolysis efficiency. The results are shown in Tables 2 and 3.

### Example 7

Compared with Example 1, the difference is that in the preparation of an organic solid-state fermentation enzyme formulation in step (II), the solid-state fermentation was performed at an ambient temperature of 36°C and a humidity of 90% for 50 h. The specific preparation method was as follows:

### (I) Preparation of solid-state seeds

The preparation method was the same as in Example 1.

### (II) Preparation of an organic solid-state fermentation enzyme formulation

1000 g of organic bean cake granules with a 6-14 mesh granule size were weighed, and 80 g of composite organic plant protein powder (composed of 60 wt% organic rice protein powder and 40 wt% organic wheat protein powder) was added. Then, 950 g of water was added, mixed uniformly, and sterilized at 121°C for 50 min to obtain a solid-state fermentation medium.

The *Aspergillus oryzae* solid-state seeds were inoculated into the above solid-state fermentation medium at an inoculation amount of 0.4% (based on the weight of the medium), and sent into a koji-making disc machine for koji-making culture. Regular ventilation and turning over the culture were carried out. The ambient temperature was controlled at 36°C, and the humidity was controlled at 94% for fermentation for 50 h. Finally, low-temperature drying was carried out at 38°C to obtain an organic solid-state fermentation enzyme formulation rich in various biological enzymes.

### (III) Preparation of an organic plant protease hydrolysate

An organic plant protease hydrolysate solution and an organic plant protease hydrolysate were prepared from the above organic solid-state fermentation enzyme formulation according to the method in step (III) of Example 1.

The enzymatic activities of proteases, glucoamylase, and cellulase in the organic solid-state fermentation enzyme formulation, as well as the total nitrogen content and amino acid nitrogen content of the organic plant protease hydrolysate solution and the plant protease hydrolysate, were determined by the methods in Example 1. The conversion rate of amino acid nitrogen in the organic plant protease hydrolysate solution was used as a measurable indicator of enzymatic hydrolysis efficiency. The results are shown in Tables 2 and 3.

### Example 8

Compared with Example 1, the difference is that in the preparation of an organic solid-state fermentation enzyme formulation in step (II), the solid-state fermentation was performed at an ambient temperature of 30°C and a humidity of 90% for 50 h. The specific preparation method was as follows:

### (I) Preparation of solid-state seeds

The preparation method was the same as in Example 1.

### (II) Preparation of an organic solid-state fermentation enzyme formulation

1000 g of organic bean cake granules with a 6-14 mesh granule size were weighed, and 80 g of composite organic plant protein powder (composed of 60 wt% organic rice protein powder and 40 wt% organic wheat protein powder) was added. Then, 950 g of water was added, mixed uniformly, and sterilized at 121°C for 50 min to obtain a solid-state fermentation medium.

The *Aspergillus oryzae* solid-state seeds were inoculated into the above solid-state fermentation medium at an inoculation amount of 0.4% (based on the weight of the medium), and sent into a koji-making disc machine for koji-making culture. Regular ventilation and turning over the culture were carried out. The ambient temperature was controlled at 30°C, and the humidity was controlled at 90% for fermentation for 50 h. Finally, low-temperature drying was carried out at 38°C to obtain an organic solid-state fermentation enzyme formulation rich in various biological enzymes.

### (III) Preparation of an organic plant protease hydrolysate

An organic plant protease hydrolysate solution and an organic plant protease hydrolysate were prepared from the above organic solid-state fermentation enzyme formulation according to the method in step (III) of Example 1.

The enzymatic activities of proteases, glucoamylase, and cellulase in the organic solid-state fermentation enzyme formulation, as well as the total nitrogen content and amino acid nitrogen content of the organic plant protease hydrolysate solution and the plant protease hydrolysate, were determined by the methods in Example 1. The conversion rate of amino acid nitrogen in the organic plant protease hydrolysate solution was used as a measurable indicator of enzymatic hydrolysis efficiency. The results are shown in Tables 2 and 3.

### Comparative Example 1

Compared with Example 1, the difference is that organic bean cake powder and composite organic plant protein powder were used instead of the organic solid-state fermentation enzyme formulation for enzymatic hydrolysis to obtain an organic plant protease hydrolysate, wherein the composite organic plant protein powder composed of 80 wt% organic rice protein powder and 20 wt% organic wheat protein powder. The specific preparation method was as follows: 1000 g of organic bean cake powder and 80 g of composite organic plant protein powder were weighed, respectively, and added with water to prepare a solution with a dry matter concentration of 16 wt%. The solution was refined into emulsion using a colloid mill, and 0.2% Angel compound protease and 0.3% papain were added, based on the dry matter weight, for enzymatic hydrolysis at a temperature of 50°C. The pH was maintained between 5.0 and 6.5 using a sodium carbonate solution. The enzymatic hydrolysis was carried out for 12 h to obtain an organic plant protease hydrolysate solution. The Angel compound protease included 40% by weight of neutral protease, 20% by weight of acidic protease, 10% by weight of glucanase, and 10% by weight of cellulase.

Perlite was added to the above organic plant protease hydrolysate solution, mixed uniformly, and then filtered using a plate-and-frame filter press to obtain a filtrate. The filtrate was concentrated under reduced pressure to obtain a concentrated solution with a dry matter concentration of 25%. Finally, 10 wt% organic maltodextrin (based on the mass of the concentrated solution) was added to the concentrated solution, stirred for dissolution, and then spray dried to obtain an organic plant protease hydrolysate.

The total nitrogen content and amino acid nitrogen content of the organic plant protease hydrolysate solution and the plant protease hydrolysate were determined by the methods in Example 1. The conversion rate of amino acid nitrogen in the organic plant protease hydrolysate solution was used as a measurable indicator of enzymatic hydrolysis efficiency. The results are shown in Table 3.

**Table 2. Enzyme activities of biological enzymes in organic solid-state fermentation enzyme formulations**

| | Enzyme activity of acidic protease (u/g) | Enzyme activity of neutral protease (u/g) | Enzyme activity of alkaline protease (u/g) | Enzyme activity of glucoamylase (u/g) | Enzyme activity of cellulase (u/g) |
|---|---|---|---|---|---|
| Example 1 | 1504 | 4863 | 2910 | 1872 | 1211 |
| Example 2 | 953 | 1752 | 2317 | 421 | 802 |
| Example 3 | 2792 | 2964 | 1347 | 982 | 2639 |
| Example 4 | 1386 | 4906 | 3298 | 1577 | 1301 |
| Example 5 | 1210 | 4458 | 2793 | 1623 | 1430 |
| Example 6 | 562 | 3031 | 629 | 1042 | 1328 |
| Example 7 | 391 | 1853 | 670 | 565 | 131 |
| Example 8 | 774 | 1912 | 1036 | 439 | 917 |
| Comparative Example 1 | \ | \ | \ | \ | \ |

As can be seen from Table 2, the organic solid-state fermentation enzyme formulation obtained in the inventive Examples 1-8 included neutral protease, acidic protease, alkaline protease, glucoamylase, and cellulase, wherein the enzyme activity of the acidic protease was 391-2792 u/g, the enzyme activity of the neutral protease was 1752-4906 u/g, the enzyme activity of the alkaline protease was 629-3298 u/g, the enzyme activity of the glucoamylase was 421-1872 u/g, and the enzyme activity of the cellulase was 131-2639 u/g. Compared with Example 6, the medium in Example 1 contained plant protein powder, and the enzyme activities of various enzymes were much greater than those of Example 6. Compared with Examples 7 and 8, the solid-state temperature shift fermentation was used in Example 1, and the enzyme activities of various enzymes were also greater than those of Examples 7 and 8.

**Table 3. Technical indicators of the organic plant protease hydrolysate solution and the protease hydrolysate**

| | Protease hydrolys ate total nitrogen g/100 g | Protease hydrolysate amino acid nitrogen g/100 g | Protease hydrolysate solution total nitrogen g/100 g | Protease hydrolysate solution amino acid nitrogen g/100 g | Conversion rate of amino acid nitrogen % |
|---|---|---|---|---|---|
| Example 1 | 11.31 | 7.25 | 1.48 | 1.02 | 68.92 |
| Example 2 | 11.22 | 6.23 | 1.41 | 0.87 | 61.70 |
| Example 3 | 12.26 | 7.48 | 1.45 | 0.96 | 66.21 |
| Example 4 | 9.27 | 6.03 | 1.22 | 0.88 | 72.13 |
| Example 5 | 10.02 | 7.11 | 1.24 | 0.91 | 73.38 |
| Example 6 | 11.28 | 5.75 | 1.45 | 0.80 | 55.17 |
| Example 7 | 11.54 | 4.59 | 1.45 | 0.63 | 43.44 |
| Example 8 | 11.31 | 6.22 | 1.44 | 0.87 | 60.42 |
| Comparati ve Example 1 | 11.97 | 5.58 | 1.18 | 0.28 | 23.72 |

As can be seen from Table 3, the conversion rate of amino acid nitrogen in the organic plant protease hydrolysate solution obtained in the inventive Examples 1-8 was 43.44-73.38%, while the conversion rate of amino acid nitrogen in the protease hydrolysate solution obtained in Comparative Example 1 by directly hydrolyzing enzymatically plant raw materials using an additional enzyme was only 23.72%. Compared with Example 6, the medium in Example 1 contained plant protein powder, the conversion rate of amino acid nitrogen was 68.92%, and the enzymatic hydrolysis efficiency was increased by nearly 25% compared to Example 6. Compared with Examples 7 and 8, the solid-state temperature shift fermentation was used in Example 1, and the enzymatic hydrolysis efficiency was increased by nearly 60% and 14% compared to Examples 7 and 8, respectively.

Finally, it is noted that the preferred examples above are only intended to illustrate rather than limit the technical solutions of the present invention. Although the present invention has been described in detail by the preferred examples as described above, it will be understood by those skilled in the art that various changes may be made in form and details without departing from the scope of the claims of the present invention.

## Claims

1. An organic solid-state fermentation enzyme formulation, the organic solid-state fermentation enzyme formulation being prepared by solid-state fermentation of protease-producing microorganisms, wherein the organic solid-state fermentation enzyme formulation comprises neutral protease, acidic protease, alkaline protease, glucoamylase, and cellulase.

2. The organic solid-state fermentation enzyme formulation according to claim 1, wherein the enzyme activity of the acidic protease is >390 u/g, the enzyme activity of the neutral protease is >1500 u/g, the enzyme activity of the alkaline protease is >600 u/g, the enzyme activity of the glucoamylase is >400 u/g, and the enzyme activity of the cellulase is >130 u/g.

3. The organic solid-state fermentation enzyme formulation according to claim 2, wherein the enzyme activity of the acidic protease is >800 u/g, the enzyme activity of the neutral protease is >4000 u/g, the enzyme activity of the alkaline protease is >1000 u/g, the enzyme activity of the glucoamylase is >1500 u/g, and the enzyme activity of the cellulase is >300 u/g.

4. The organic solid-state fermentation enzyme formulation according to claim 3, wherein the enzyme activity of the acidic protease is >1200 u/g, the enzyme activity of the alkaline protease is >2200 u/g, and the enzyme activity of the cellulase is >1200 u/g.

5. The organic solid-state fermentation enzyme formulation according to any one of claims 1-4, wherein the protease-producing microorganisms comprise one or a combination of two or more selected from the group consisting of *Aspergillus oryzae, Aspergillus niger,* and *Bacillus sp.,* preferably *Aspergillus oryzae* and *Bacillus sp.;* preferably, the weight ratio of *Aspergillus oryzae* to *Bacillus sp.* is (70-90):(10-30).

6. A preparation method for the organic solid-state fermentation enzyme formulation according to any one of claims 1-5, comprising the steps of:
(1) performing seed culture of protease-producing microorganisms to obtain solid-state seeds; and
(2) performing solid-state fermentation of the solid-state seeds obtained in step (1) to obtain an organic solid-state fermentation enzyme formulation.

7. The preparation method according to claim 6, wherein the solid-state fermentation is solid-state temperature shift fermentation, which is first carried out at 36-40°C for 6-20 h and then at 28-32°C for 30-50 h; preferably, the solid-state temperature shift fermentation is first carried out at 36-38°C for 8-15 h and then at 30-32°C for 40-44 h.

8. The preparation method according to claim 6 or 7, wherein both the seed medium in step (1) and the solid-state fermentation medium in step (2) contain organic starch agricultural and sideline products and/or organic oil agricultural and sideline products; preferably, both the seed medium in step (1) and the solid-state fermentation medium in step (2) contain organic starch agricultural and sideline products and organic oil agricultural and sideline products; more preferably, the content of the organic starch agricultural and sideline products is 9-40% by weight, and the content of the organic oil agricultural and sideline products is 60-91% by weight.

9. The preparation method according to claim 8, wherein the organic starch agricultural and sideline products comprise one or a combination of two or more selected from the group consisting of organic rice, organic wheat, and organic corn, preferably organic corn.

10. The preparation method according to claim 8 or 9, wherein the organic oil agricultural and sideline products comprise organic bean cake and/or organic rapeseed cake, preferably organic bean cake.

11. The preparation method according to any one of claims 6-10, wherein both the seed medium in step (1) and the solid-state fermentation medium in step (2) further contain organic plant protein powder; preferably, the organic plant protein powder is 6-10%, and the organic starch agricultural and sideline products and/or organic oil agricultural and sideline products is 90-94%, based on the total weight of 100% of the medium.

12. The preparation method according to claim 11, wherein the organic plant protein powder comprises organic rice protein powder and/or organic wheat protein powder; preferably, the organic plant protein powder comprises 60-80% by weight of organic rice protein powder and 20-40% by weight of organic wheat protein powder.

13. The preparation method according to any one of claims 6-12, wherein the inoculation amount for the seed culture in step (1) is 0.3-0.5% based on the weight of the seed medium.

14. The preparation method according to any one of claims 6-13, wherein the inoculation amount for the solid-state fermentation in step (2) is 0.3-0.5% based on the weight of the solid-state fermentation medium.

15. The preparation method according to any one of claims 6-14, further comprising the step of drying the organic solid-state fermentation enzyme formulation; preferably, the drying temperature is 37-40°C.

16. A preparation method for an organic plant protease hydrolysate, comprising hydrolyzing enzymatically the organic solid-state fermentation enzyme formulation according to any one of claims 1-5 or an organic solid-state fermentation enzyme formulation obtained by the preparation method according to any one of claims 6-13 using an additional enzyme.

17. The preparation method according to claim 16, wherein the additional enzyme is used in an amount of 0.2-0.8% based on the dry matter weight of the organic solid-state fermentation enzyme formulation; preferably, the additional enzyme comprises one or a combination of two or more selected from the group consisting of papain, bromelain, neutral protease, acidic protease, alkaline protease, glucanase, and cellulase; more preferably, the additional enzyme comprises 0.1-0.3% papain, 0.04-0.08% neutral protease, 0.02-0.04% acidic protease, 0.01-0.02% glucanase, and 0.01-0.02% cellulase, based on the dry matter weight of the organic solid-state fermentation enzyme formulation.

18. The preparation method according to claim 16 or 17, wherein the enzymatic hydrolysis temperature is 45-55°C, and the enzymatic hydrolysis time is 10-15 h; preferably, the enzymatic hydrolysis pH is between 4.5 and 7.5.

19. The preparation method according to any one of claims 16-18, further comprising the step of refining the organic solid-state fermentation enzyme formulation before the enzymatic hydrolysis; preferably, the dry matter content of a slurry during refining is 12-16% by weight.

20. The preparation method according to any one of claims 16-19, further comprising the steps of sequentially filtering, concentrating, and drying the enzymatic hydrolysate.

21. An organic plant protease hydrolysate obtained by the preparation method according to any one of claims 16-20; preferably, the total nitrogen content is >9%, and the amino acid nitrogen content is >4.5%, based on the dry matter weight of the organic plant protease hydrolysate.

22. Use of an organic plant protease hydrolysate obtained by the preparation method according to any one of claims 16-21 or the organic plant protease hydrolysate according to claim 22 as an organic nitrogen source in the fermentation field of yeast, lactic acid bacteria, or *Bacillus sp.*

23. Use of an organic plant protease hydrolysate obtained by the preparation method according to any one of claims 16-21 or the organic plant protease hydrolysate according to claim 22 as an organic tasty agent or seasoning base in the field of food seasoning.

24. Use of the organic solid-state fermentation enzyme formulation according to any one of claims 1-5 or an organic solid-state fermentation enzyme formulation obtained by the preparation method according to any one of claims 6-15 as an organic protease for the enzymatic hydrolysis of plant proteins.
